# EUROPEAN PATENT APPLICATION

(11) **EP 4 647 497 A1**
(43) Date of publication of application: **12.11.2025**
(21) Application number: 24781175.5
(22) Date of filing: 26.03.2024
(51) Int. Cl.: C12N 15/70, C12N 9/10, C12P 13/06, C12P 13/12, C07K 14/245

(54) **RECOMBINANT MICROORGANISM IN WHICH ACTIVITY OF MANGANESE TRANSPORT PROTEIN IS REGULATED, O-PHOSPHOSERINE USING SAME, CYSTEINE, AND METHOD FOR PRODUCING DERIVATIVE THEREOF**

(30) Priority: 31.03.2023 KR 20230043073
(71) Applicant: CJ CheilJedang Corporation, Seoul 04560 (KR)
(72) Inventor: SIM, Hee-jin, Seoul 04560 (KR); RHO, Jin Ah, Seoul 04560 (KR); JUNG, Hwi-Min, Seoul 04560 (KR); CHO, Hyunjin, Seoul 04560 (KR)
(74) Representative: Meissner Bolte Partnerschaft mbB
(86) International application number: PCT/KR2024/003753
(87) International publication number: WO 2024/205184

(57) **Abstract**

The present disclosure relates to a recombinant microorganism, in which the activity of a protein having manganese importing activity is regulated, and a method for producing O-phosphoserine, cysteine or cysteine derivatives using the microorganism.

## Description

### [Technical Field]

The present disclosure relates to a recombinant microorganism with regulated activity of manganese import protein, and a method for producing O-phosphoserine, cysteine, and cysteine derivatives using the same.

### [Background Art]

L-cysteine, an amino acid playing an important role in sulfur metabolism in all living organisms, is used not only in the synthesis of biological proteins such as hair keratin, *etc*., glutathione, biotin, methionine, and other sulfur-containing metabolites, but also as a precursor for biosynthesis of coenzyme A.

Methods of producing L-cysteine using microorganisms known in the art include: 1) a method of biologically converting D,L-2-aminothiazoline-4-carboxylic acid (D,L-ATC) into L-cysteine using microorganisms, 2) a method of producing L-cysteine by direct fermentation using *E. coli* (EP0885962B; Wada M and Takagi H, Appl. Microbiol. Biochem., 73:48-54, 2006), and 3) a method of producing O-phosphoserine (hereinafter "OPS") by fermentation using microorganisms, and then converting O-phosphoserine into L-cysteine by reacting O-phosphoserine with a sulfide under the catalytic action of O-phosphoserine sulfhydrylase (hereinafter "OPSS") (US 8557549 B2), *etc.*

In particular, in order to produce cysteine by the method 3) at high yield, OPS, the precursor, should be produced in excessive amounts.

### [Disclosure]s

### [Technical Problem]

The technical problem of the present disclosure is to provide a recombinant microorganism, in which the activity of a protein having manganese importing activity is regulated, and a method for producing O-phosphoserine, cysteine, and cysteine derivatives using the same.

### [Technical Solution]

It is one object of the present disclosure to provide an O-phosphoserine-producing recombinant microorganism, in which the activity of MntH protein is enhanced compared to the endogenous activity.

It is another object of the present disclosure to provide a method for producing O-phosphoserine using the O-phosphoserine-producing recombinant microorganism of the present disclosure.

It is still another object of the present disclosure to provide a method for producing cysteine or cysteine derivatives using the O-phosphoserine-producing recombinant microorganism of the present disclosure.

### [Advantageous Effects]

When O-phosphoserine is produced using the O-phosphoserine-producing recombinant microorganism of the present disclosure, in which the activity of MntH protein is enhanced compared to the endogenous activity, O-phosphoserine can be produced with high yield compared to when using an existing non-modified strain.

### [Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below. Additionally, a number of papers and patent documents have been cited throughout the present specification. The content of the cited papers and patent documents is incorporated herein by reference in their entirety and the level of technical field to which the present disclosure belongs and the contents of the present disclosure will be described more clearly.

One aspect of the present disclosure provides an O-phosphoserine-producing recombinant microorganism, in which the activity of MntH protein is enhanced compared to the endogenous activity.

As used herein, the term "O-phosphoserine (hereinafter, "OPS")" refers to a phosphoric acid ester of serine which serves as a constituting component for many proteins. The OPS is a precursor of L-cysteine and can be converted to cysteine by reacting with a sulfide under the catalytic action of OPS sulfhydrylase (hereinafter "OPSS"), but is not limited thereto (US 2012-0190081 A1).

As used herein, the term "MntH protein" is a protein with divalent ion transporter activity, which is classified into the NRAMP family, and in one example, it may be an Mn²⁺ uptake NRAMP. As used herein, the term "Mn²⁺ uptake NRAMP (natural resistance-associated macrophage protein) transporter (MntH)" refers to a protein with the activity of a Mn²⁺/Fe²⁺: H⁺ symporter, and has the activity of importing manganese (Mn) into cells.

The amino acid sequence of MntH protein can be obtained from GenBank of NCBI, a known database, *etc.*

In one example, the MntH protein of the present disclosure may be derived from a microorganism. The microorganism may specifically be derived from a microorganism of the genus of *Escherichia,* but is not limited thereto.

In another example, the amino acid sequence of the MntH protein of the present disclosure may be WP000186369.1 or EEW8215783.1 derived from *Escherichia coli* (*E. coli*), but it is obvious that it may include proteins with MntH protein activity from various origins.

In the present disclosure, the MntH protein may have, include, or consist of an amino acid sequence of SEQ ID NO: 1, or may consist essentially of the amino acid sequence above.

In the present disclosure, the MntH protein may include an amino acid sequence having at least 70% or more, 75% or more, 76% or more, 85% or more, 90% or more, 95% or more, 96% or more, 97% or more, 98% or more, 99% or more, 99.5% or more, 99.7% or more or 99.9% or more homology or identity to the amino acid sequence of SEQ ID NO: 1. Additionally, it is apparent that any protein having an amino acid sequence, in which part of the sequence is deleted, modified, substituted, conservatively substituted or added, may also fall within the scope of the present disclosure as long as the amino acid sequence has such a homology or identity, and exhibits an efficacy corresponding to the protein including the amino acid sequences of SEQ ID NO: 1. In one example, the MntH protein may be composed of 412 to 428 amino acids including the amino acid sequence of SEQ ID NO: 1

For example, it may be sequence additions that do not alter the function of the protein of the present disclosure, naturally occurring mutations, silent mutations therein, or conservative substitutions at the N-terminus, C-terminus and/or within the amino acid sequences.

As used herein, the term "conservative substitution" refers to the substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. Typically, conservative substitutions may have little or no effect on the activity of a protein or polypeptide.

As used herein, the term 'homology' or 'identity' refers to the degree of relatedness between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and can be used with a default gap penalty established by the program being used. Substantially, homologous or identical sequences are generally expected to hybridize to all or part of the sequences under moderate or highly stringent conditions. It is apparent that hybridization with polynucleotides containing general codon or degenerate codons in hybridizing polynucleotides is also included.

Whether any two polynucleotide or polypeptide sequences have homology, similarity, or identity may be determined by a known computer algorithm such as the "FASTA" program using default parameters as in Pearson et al. (1988) Proc. Natl. Acad. Sci. USA 85:2444. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (the GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, homology, similarity, or identity may be determined using BLAST or ClustalW of the National Center for Biotechnology Information.

The homology, similarity, or identity of polynucleotides or polypeptides may be determined by comparing sequence information using, for example, the GAP computer program, such as Needleman et al. (1970), *J Mol Biol.* 48:443, as disclosed in Smith and Waterman, *Adv. Appl. Math* (1981) 2:482. In summary, the GAP program defines the homology, similarity or identity as the value obtained by dividing the number of similarly aligned symbols (i.e., nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl Acids Res. 14:6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps.

The MntH protein of the present disclosure may be encoded by a *mntH* gene.

In one example, the *mntH* gene may be a polynucleotide encoding EEW8215783.1 derived from *Escherichia coli,* but is not limited thereto. In another example, the *mntH* gene may be a polynucleotide encoding WP_000186369.1 derived from *Escherichia coli,* and in still another example, it may be a sequence included in CP116188.1 derived from *Escherichia coli,* but is not limited thereto, and it is apparent that the gene may include *mntH* genes encoding proteins with MntH protein activity from various origins.

As used herein, the term "polynucleotide", which is a polymer of nucleotides composed of nucleotide monomers connected in a lengthy chain by a covalent bond, is a DNA or RNA strand having at least a certain length. More specifically, it may refer to a polynucleotide fragment encoding the protein.

The polynucleotide encoding the MntH protein of the present disclosure may include a nucleotide sequence encoding the amino acid sequence of SEQ ID NO: 1. In one example of the present disclosure, the polynucleotide of the present disclosure may have or include a nucleotide sequence of SEQ ID NO: 2. Additionally, the polynucleotide of the present disclosure may consist or consist essentially of the nucleotide sequence of SEQ ID NO: 2. Specifically, the *mntH* may be encoded by the polynucleotide represented by the nucleotide sequence of SEQ ID NO: 2.

The polynucleotide of the present disclosure may undergo various modifications in the coding region within the scope that does not change the amino acid sequences of the MntH protein, due to codon degeneracy or in consideration of the codons preferred in an organism in which the MntH protein is to be expressed. Specifically, the polynucleotide of the present disclosure may have or include a nucleotide sequence having 70% or more, 75% or more, 76% or more, 85% or more, 90% or more, 95% or more, 96% or more, 97% or more, 98% or more, or 99% or more homology or identity to the sequence of SEQ ID NO: 2, or may consist or consist essentially of a nucleotide sequence having 70% or more, 75% or more, 76% or more, 85% or more, 90% or more, 95% or more, 96% or more, 97% or more, 98% or more, or 99% or more homology or identity to the sequence of SEQ ID NO: 2, but is not limited thereto.

Additionally, the polynucleotide of the present disclosure may include a probe that may be prepared from a known gene sequence, for example, any polynucleotide sequence which may hybridize with a sequence complementary to all or part of the polynucleotide sequence of the present disclosure under stringent conditions without limitation. The "stringent conditions" refer to conditions which allow the specific hybridization between polynucleotides. Such conditions are disclosed in detail in the literature (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having high homology or identity, *i.e.*, polynucleotides having 70% or more, 75% or more, 76% or more, 85% or more, 90% or more, 95% or more, 96% or more, 97% or more, 98% or more, or 99% or more homology or identity hybridize with each other, while polynucleotides having homology or identity lower than the above homology or identity do not hybridize with each other; or may include ordinary washing conditions of Southern hybridization, i.e., washing once, specifically twice or three times, at a salt concentration and a temperature corresponding to 60°C., 1×SSC, 0.1% SDS, specifically 60°C., 0.1×SSC, 0.1% SDS, and more specifically 68°C., 0.1×SSC, 0.1% SDS.

Hybridization requires that two nucleic acids have complementary sequences, although mismatches between bases are possible depending on the stringency of the hybridization. The term "complementary" is used to describe a relationship between nucleotide bases that may hybridize with each other. For example, with respect to DNA, adenine is complementary to thymine, and cytosine is complementary to guanine. Therefore, the polynucleotide of the present disclosure may also include an isolated nucleic acid fragment complementary to the entire sequence as well as a nucleic sequence substantially similar thereto.

Specifically, polynucleotides having a homology or identity to the polynucleotide of the present disclosure may be detected using the hybridization conditions including a hybridization step at a Tₘ value of 55 °C under the above-described conditions. Further, the Tₘ value may be 60 °C, 63 °C, or 65 °C, but is not limited thereto, and may be appropriately adjusted by those skilled in the art depending on the purpose thereof.

The appropriate stringency for hybridizing the polynucleotides depends on the length and degree of complementarity of the polynucleotides, and these variables are well known in the art (*e.g*., Sambrook et al., supra).

As used herein, the term "vector" refers to a DNA construct containing a nucleotide sequence encoding the target protein operably linked to a suitable expression regulatory sequence so as to be able to express the target protein in a suitable host cell. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

The vector used in the present disclosure is not particularly limited as long as it can be replicated in a host cell, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pBR, pUC, pBluescriptll, pGEM, pTZ, pCL, and pET, *etc*. may be used. Specifically, pSKH130 (US Application Publication No. 2020-0048619), pSK, pDZ, pDC, pDCM2, pDC24, pACYC177, pACYC184, pCL, pECCG117, pUC19, pBR322, pMW118, pCC1BAC vector, *etc.* may be used.

The insertion of the polynucleotide into the chromosome may be performed by any method known in the art, for example, homologous recombination, but is not limited thereto.

As used herein, the term "transformation" refers to the introduction of a recombinant vector containing a polynucleotide encoding a target protein into a host cell so that the protein encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. The method for transforming the vector includes any method of introducing a nucleic acid into a cell, and may be performed by selecting a suitable standard technique as known in the art depending on the host cell. For example, the transformation may be carried out via electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, *etc.,* but the method is not limited thereto.

Further, as used herein, the term "operably linked" means that the polynucleotide sequence is functionally linked to a promoter sequence or expression regulatory region that initiates and mediates transcription of the polynucleotide encoding the target protein of the present disclosure. The operable linkage may be prepared using a genetic recombinant technology known in the art, and site-specific DNA cleavage and linkage may be prepared using cleavage and linking enzymes, *etc.,* known in the art, but is not limited thereto.

The microorganism of the present disclosure is not limited by its type as long as it can produce OPS, and may be any prokaryotic or eukaryotic microorganism, and specifically, a prokaryotic microorganism. In one example, it may include microbial strains belonging to the genus *Escherichia,* the genus *Erwinia*, the genus *Serratia*, the genus *Providencia*, the genus *Corynebacterium* and the genus *Brevibacterium*, and specifically, a microorganism belonging to the genus *Escherichia,* and more specifically, *Escherichia coli,* but is not limited thereto. For example, in the case of the microorganism belonging to the genus *Escherichia,* OPS and L-serine can be produced through SerA, SerC and SerB, which are enzymes of the biosynthetic pathway of L-serine (Ahmed Zahoor, Computational and structural biotechnology journal, vol 3, 2012 October; Wendisch V F et al., Curr Opin Microbiol. 2006 Jun;9(3):268-74; Peters-Wendisch P et al., Appl Environ Microbiol. 2005 Nov;7 1(II):7 139-44.).

As used herein, the term "O-phosphoserine (OPS)-producing microorganism" refers to a microorganism having the ability to naturally produce O-phosphoserine or a microorganism in which the O-phosphoserine-producing ability has been imparted to a parent strain having no ability to produce O-phosphoserine. Specifically, the microorganism may be an OPS-producing microorganism with an enhanced MntH protein activity due to natural or artificial genetic modification. For the purpose of the present disclosure, the OPS-producing microorganism may be any microorganism capable of producing O-phosphoserine by enhancing the activity of the MntH protein by the method disclosed in the present disclosure. As used herein, the "O-phosphoserine (OPS)-producing microorganism" may be used interchangeably with "microorganism producing O-phosphoserine (OPS)" or "microorganism having an O-phosphoserine-producing ability".

In one embodiment, the OPS-producing microorganism of the present disclosure may be a genetically modified microorganism or a recombinant microorganism, in which the activity of the MntH protein is enhanced, thereby increasing the desired OPS-producing ability, but is not limited thereto. The recombinant microorganism may be a microorganism in which the O-phosphoserine-producing ability is enhanced compared to the endogenous O-phosphoserine-producing ability.

The microorganism of the present disclosure may have an increased MntH protein activity compared to the endogenous activity. Specifically, the microorganism of the present disclosure may be a microorganism in which the MntH protein or the *mntH* gene encoding the same is enhanced; or a microorganism (*e.g.*, a recombinant microorganism) that has been genetically modified to enhance the MntH protein or the *mntH* gene encoding the same, but is not limited thereto.

As used herein, the term "enhancement" of a polypeptide (*e.g*., proteins specified by the name of each enzyme) means that the activity of a polypeptide is increased compared to its endogenous activity. The enhancement may be used interchangeably with terms such as activation, up-regulation, overexpression, increase, *etc.* In particular, the activation, enhancement, up-regulation, overexpression and increase may include both cases in which an activity not originally possessed is exhibited, or the activity is enhanced compared to the endogenous activity or the activity before modification. The "endogenous activity" refers to the activity of a particular polypeptide originally possessed by a parent strain before transformation or a non-modified microorganism, when a trait is altered through genetic modification caused by natural or artificial factors, and may be used interchangeably with "activity before modification". The "enhancement", "up-regulation", "overexpression" or "increase" in the activity of a polypeptide compared to its endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of the polypeptide may be confirmed by the increase in the level of activity of the polypeptide, expression level, or the amount of product excreted from the polypeptide.

For the purpose of the present disclosure, the microorganism of the present disclosure has an enhanced MntH protein activity, thereby enhancing OPS-producing ability. The non-modified microorganism in which the MntH protein is not enhanced, which is the target strain for comparing the increase in the OPS-producing ability or the MntH protein, may be CA07-0012 (KCCM 11121P, US 8557549 B2), which is an OPS-producing strain, in which the endogenous phosphoserine phosphatase (SerB) has been deleted and thus weakened the OPS-decomposing ability, the strain (CA07-4821 of the present disclosure), in which the expression of YhhS having an OPS-exporting ability is enhanced in the CA07-0012 strain, and the strain (CA07-4828 of the present disclosure), in which the activity of NADH:quinone oxidoreductase is further enhanced in the CA07-4821 strain, but is not limited thereto.

The enhancement of the activity of the polypeptide may be applied by various methods well known in the art, and the method is not limited as long as it can enhance the activity of the target polypeptide compared to that of a microorganism before modification. Specifically, genetic engineering and/or protein engineering well known to those skilled in the art, which is the common method of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc*.*).

Specifically, the enhancement of the activity of the polypeptide of the present disclosure may be achieved by:
1) increasing the intracellular copy number of a polynucleotide encoding the polypeptide;
2) modifying the expression regulatory sequence of a gene encoding the polypeptide on the chromosome;
3) modifying the nucleotide sequence encoding the initiation codon or 5'-UTR of the gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e.g*., modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of the polynucleotide encoding the polypeptide;
8) analyzing the tertiary structure of the polypeptide and thereby selecting and modifying the exposed site, or chemically modifying the same;
9) regulating the cellular localization of the polypeptide; or
10) a combination of two or more selected from above 1 to 9), but is not particularly limited thereto.

More specifically,
The 1) method of increasing the intracellular copy number of a polynucleotide encoding the polypeptide may be achieved by introducing a vector, which is operably linked to the polynucleotide encoding the polypeptide and is able to replicate and function regardless of the host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto.

The 2) method of replacing the expression regulatory region (or expression regulatory sequence) of a gene encoding the polypeptide on the chromosome with a sequence having a strong activity may be achieved, for example, by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome-binding site, and a sequence regulating the termination of transcription and translation, *etc.* The method may specifically include inserting a strong promoter downstream of the original promoter, but is not limited thereto.

Examples of the known strong promoter may include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, rmf promoter, serC promoter, *etc.*, but the strong promoter is not limited thereto.

The 3) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or a polynucleotide sequence modified to have a stronger activity, or an amino acid sequence or a polynucleotide sequence modified to enhance the activity, but are not limited thereto. The replacement may specifically be performed by inserting the polynucleotide into the chromosome by homologous recombination, but is not limited thereto.

The vector used herein may further include a selection marker to confirm the insertion into the chromosome. The selection marker is for selecting the cells transformed with the vector, that is, for confirming the insertion of the gene to be introduced, and markers that provide selectable phenotypes, such as drug resistance, auxotrophy, resistance to cell toxic agents, or expression of surface proteins, may be used, but the markers are not limited thereto. Additionally, only cells expressing the selection marker are able to survive or to show different phenotypes under the environment treated with the selective agent, and thus the transformed cells may be selected.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables the production of the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

The 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

The 9) method of regulating the cellular localization of the polypeptide may be achieved by targeting the polypeptide to a particular organelle or a particular space within the cell. For example, it may be achieved by targeting the polypeptide to the periplasm or cytoplasm through addition or removal of a leader sequence that functions in targeting polypeptides, but is not limited thereto.

In one embodiment, the enhancement of the activity of the MntH protein may be achieved by modifying the expression regulatory region of a gene encoding the polypeptide on the chromosome of 2) above.

In any one of the above-described embodiments, the enhancement of the activity of the MntH protein of the present disclosure may be an increase in the expression of a gene encoding the MntH protein. In any one of the above-described embodiments, the enhancement of the activity of the MntH protein may include a gene expression regulatory sequence with enhanced activity in the upstream of the gene encoding the same. Specifically, the upstream of the gene encoding the MntH protein may be the upstream of the *mntH* gene. In one embodiment, the enhancement of the activity of the MntH protein may be an enhancement of the expression of the gene sequence by modifying the expression regulatory sequence of the *mntH* gene. Specifically, the modification of the expression regulatory sequence may be an additional insertion of a gene expression regulatory sequence with enhanced activity between the endogenous promoter of the *mntH* gene and the *mntH* gene, for example, the gene expression regulatory sequence may be a promoter, but is not limited thereto.

Such enhancement of the polypeptide activity may mean that the activity or concentration of the corresponding polypeptide is increased relative to the activity or concentration of the polypeptide expressed in a wild-type strain or a microbial strain before modification, or that the amount of product produced from the polypeptide is increased, but is not limited thereto.

As used herein, the term "strain before modification" or "microorganism before modification" does not exclude a strain containing a mutation that may occur naturally in a microorganism, and may refer to a natural-type strain itself, or a strain before the trait is altered due to genetic modification caused by natural or artificial factors. In the present disclosure, the modification of the traits may be an enhancement of the activity of MntH protein. The "strain before modification" or "microorganism before modification" may be used interchangeably with "non-mutant strain", "non-modified strain", "non-mutant microorganism", "non-modified microorganism" or "reference microorganism".

The modification of a part or all of the polynucleotide in the microorganism of the present disclosure may be achieved by (a) homologous recombination using a vector for chromosomal insertion in the microorganism or genome editing using an engineered nuclease (e.g., CRISPR-Cas9), and/or (b) being induced by light, such as ultraviolet rays and radiation, *etc.* and/or chemical treatments, but is not limited thereto. The method of modifying a part or all of the gene may include a method using DNA recombination technology. For example, a part or all of the gene may be deleted by injecting a nucleotide sequence or a vector containing a nucleotide sequence homologous to the target gene into the microorganism to induce homologous recombination. The injected nucleotide sequence or the vector may include a dominant selection marker, but is not limited thereto.

The microorganism of the present disclosure may be a microorganism having an enhanced OPS-producing ability.

For the purpose of the present disclosure, the recombinant microorganism of the present disclosure may be a microorganism having an increased OPS-producing ability by enhancing the MntH protein or a polynucleotide encoding the same in a natural wild-type microorganism; or an OPS-producing microorganism, which includes the MntH protein or a polynucleotide encoding the protein, compared to the natural wild-type microorganism or the OPS-producing microorganism, which includes the MntH protein or a polynucleotide encoding the protein, but is not limited thereto. For example, the natural wild-type microorganism or the OPS-producing microorganism, which includes the MntH protein or a polynucleotide encoding the protein, may be the target strain for comparing the increase in the OPS-producing ability or the activity of the MntH protein, but is not limited thereto.

In one example, the recombinant strain having an increased OPS-producing ability may have an increased OPS-producing ability by about 1% or more, specifically, about 1.7% or more, 2% or more, about 2.3% or more, about 3% or more, about 4% or more, about 5% or more, about 5.3% or more, about 6% or more, about 7% or more, about 8% or more, about 9% or more, or about 10% or more (the upper limit is not particularly limited, for example, about 200% or less, about 150% or less, about 100% or less, about 50% or less, about 40% or less, about 30% or less, about 20% or less or about 15% or less) as compared to the OPS-producing ability of a parent strain before modification or a non-modified microorganism, but is not limited thereto, as long as it has an increased + value compared to the production ability of a parent strain before modification or a non-modified microorganism. In another example, the microorganism having an increased OPS-producing ability may have an increased OPS-producing ability by about 1.01 times or more, about 1.017 times or more, about 1.02 times or more, about 1.023 times or more, about 1.03 times or more, about 1.04 times or more, about 1.05 times or more, about 1.053 times or more, about 1.06 times or more, about 1.07 times or more, about 1.08 times or more, about 1.09 times or more or about 1.10 times or more (the upper limit is not particularly limited, for example, about 10 times or less, about 5 times or less, about 3 times or less, or about 2 times or less) as compared to that of a parent strain before modification or a non-modified microorganism, but is not limited thereto.

The production ability may be evaluated by measuring the production amount of the desired product obtained by culturing in a medium. The evaluation can measure the production amount of the desired product using a suitable method known in the art. For example, HPLC (High Performance Liquid Chromatography), GC (Gas Chromatography), GC/MS (Gas Chromatography-Mass Spectrometry), LC/MS (Liquid chromatography-mass spectrometry), GPC (Gel Permeation Chromatography), or a combination thereof may be used, and the production amount of the desired product may be measured using suitable methods known in the art.

The microorganism of the present disclosure may further enhance the ability to produce OPS and/or the ability to export the OPS out of the cell; or may include modifications that enhance OPS-decomposing ability and/or importing ability.

Examples of the modifications that enhance the ability to produce OPS and/or the ability to export OPS out of the cell; or enhance the OPS-decomposing and/or importing ability may include enhancement of the activity of NADH:quinone oxidoreductase; attenuation of the activity of phosphoserine phosphotase (SerB); enhancement of the activity of phosphoserine export protein (YhhS); or a combination of these modifications, but are not limited thereto.

As used herein, the term "weakening" of the activity of a polypeptide is a comprehensive concept including both reduced or no activity compared to its endogenous activity. The weakening may be used interchangeably with terms such as inactivation, deficiency, down-regulation, decrease, reduce, attenuation, *etc*.

The weakening may also include a case where the polypeptide activity itself is decreased or removed compared to the activity of the polypeptide originally possessed by a microorganism due to mutation of a polynucleotide encoding the polypeptide; a case where the overall level of intracellular polypeptide activity and/or concentration (expression level) is decreased compared to a natural strain due to the inhibition of expression of a gene of a polynucleotide encoding the polypeptide, or the inhibition of translation into the polypeptide, *etc.*; a case where the polynucleotide is not expressed at all; and/or a case where no polypeptide activity is observed even when the polynucleotide is expressed. As used herein, the term "endogenous activity" refers to the activity of a particular polypeptide originally possessed by a parent strain before transformation, a wild-type or a non-modified microorganism, when a trait is altered through genetic modification caused by natural or artificial factors, and may be used interchangeably with "activity before modification". The expression that the polypeptide activity is "weakened, inactivated, deficient, decreased, down-regulated, reduced or attenuated" compared to its endogenous activity means that the polypeptide activity is decreased compared to the activity of a particular polypeptide originally possessed by a parent strain before transformation or a non-modified microorganism.

The weakening of the polypeptide activity may be performed by any method known in the art, but the method is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc*.*).

Specifically, the weakening of the polypeptide activity of the present disclosure may be achieved by:
1) deleting a part or all of a gene encoding the polypeptide;
2) modifying the expression regulatory region (expression regulatory sequence) such that the expression of a gene encoding the polypeptide is decreased;
3) modifying the amino acid sequence constituting the polypeptide such that the polypeptide activity is removed or weakened (*e.g*., deletion/substitution/addition of one or more amino acids on the amino acid sequence);
4) modifying the gene sequence encoding the polypeptide such that the polypeptide activity is removed or weakened (*e.g*., deletion/substitution/addition of one or more nucleotides on the nucleotide sequence of the polypeptide gene to encode a polypeptide that has been modified to remove or weaken the activity of the polypeptide);
5) modifying the nucleotide sequence encoding the initiation codon, the Shine-Dalgarno (SD) sequence, or 5'-UTR of the gene transcript encoding the polypeptide;
6) introducing an antisense oligonucleotide (*e.g*., antisense RNA), which binds complementarily to the gene transcript encoding the polypeptide;
7) adding a sequence complementary to the Shine-Dalgarno (SD) sequence on the front end of the SD sequence of the gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of the gene sequence encoding the polypeptide;
9) regulating the cellular localization of the polypeptide; or
10) a combination of two or more selected from the methods 1) to 9) above, but is not particularly limited thereto.

For example,
The 1) method of deleting a part or all of the gene encoding the polypeptide may be achieved by deleting all of the polynucleotide encoding the endogenous target polypeptide within the chromosome, or by replacing the polynucleotide with a polynucleotide having a partially deleted nucleotide, or with a marker gene.

The 2) method of modifying the expression regulatory region (expression regulatory sequence) may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome-binding site, and a sequence for regulating the termination of transcription and translation, but is not limited thereto.

The 3) and 4) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide, or by replacing the sequence with an amino acid sequence or a polynucleotide sequence modified to have a weaker activity, or an amino acid sequence or a polynucleotide sequence modified to have no activity, but are not limited thereto. For example, the expression of the gene may be inhibited or weakened by introducing a mutation into the polynucleotide sequence to form a termination codon, but is not limited thereto.

The 5) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a lower polypeptide expression rate than the endogenous initiation codon, but is not limited thereto.

The 6) method of introducing an antisense oligonucleotide (*e.g*., antisense RNA), which binds complementary to the gene transcript encoding the polypeptide, can be found in the literature [Weintraub, H. et al., Antisense-RNA as a molecular tool for genetic analysis, Reviews - Trends in Genetics, Vol. 1(1) 1986].

The 7) method of adding a sequence complementary to the Shine-Dalgarno (SD) sequence on the front end of the SD sequence of the gene encoding the polypeptide to form a secondary structure, thereby inhibiting the ribosome attachment may be achieved by inhibiting mRNA translation or reducing the speed thereof.

Further, the 8) reverse transcription engineering (RTE), which adds a promoter, which is to be reversely transcribed, on the 3' terminus of the open reading frame (ORF) of the gene sequence encoding the polypeptide, may be achieved by forming an antisense nucleotide complementary to the gene transcript encoding the polypeptide to weaken the activity.

The 9) method of regulating the cellular localization of the polypeptide may be achieved by targeting the polypeptide to a particular organelle or a particular space within the cell. For example, it may be achieved by targeting the polypeptide to the periplasm or cytoplasm through addition or removal of a leader sequence that functions in targeting polypeptides, but is not limited thereto.

Such weakening of the polypeptide activity may mean that the activity or concentration of the corresponding polypeptide is decreased relative to the activity or concentration of the polypeptide expressed in a wild-type strain or a microbial strain before modification, or that the amount of product produced from the polypeptide is decreased, but is not limited thereto.

In one embodiment, the recombinant microorganism of the present disclosure may be a microorganism in which the activity of one or more selected from NADH:quinone oxidoreductase and O-phosphoserine export protein is further enhanced compared to the endogenous activity.

In any one of the above-described embodiments, the recombinant microorganism of the present disclosure may be a microorganism in which the activity of NADH:quinone oxidoreductase is enhanced.

As used herein, the term "NADH:quinone oxidoreductase (hereinafter "Nuo")" is an enzyme that reduces quinone in the cell membrane by oxidizing NADH in the electron transport chain of microorganisms. The enzyme protein may also be named NADH dehydrogenase-1 (NDH-1). The gene encoding the protein may be, for example, the nuoABCEFGHIJKLMN gene cluster, but is not limited thereto. The nuoABCEFGHIJKLMN gene cluster constitutes the *nuo* operon, and the expression thereof can be regulated by a promoter in front of the operon and a polynucleotide in the ribosome binding site. In the present disclosure, the 'nuoABCEFGHIJKLMN gene' may be used interchangeably with 'gene encoding NADH:quinone oxidoreductase, 'nuoABCEFGHIJKLMN gene', '*nuo* operon', and '*nuo* gene'.

The Nuo is a complex of 13 subunit proteins (NuoA, NuoB, NuoC, NuoE, NuoF, NuoG, NuoH, Nuol, NuoJ, NuoK, NuoL, NuoM, NuoN). The two types of operons, nuoABCEFGHIJKL and nuoMN, are used as templates in the translation of each subunit protein. The structure of the *nuo* operon can be found in EcoCyc (www.biocyc.org) (Accession No.: EG12082). The *nuo* operon is known to include a structure gene and an expression regulatory region. The "expression regulatory region" of the *nuo* operon refers to a region that exists upstream of the structure gene constituting the *nuo* operon and thus can regulate the expression of the structure gene. The expression regulatory region of the *nuo* operon may include a promoter (nuoA promoter and/or nuoM promoter) excluding the structure gene and an operator, and may specifically include a promoter.

The operon is as described above.

In one embodiment, the enhancement of the activity of the NADH:quinone oxidoreductase may be achieved by modification such that the activity of the NADH:quinone oxidoreductase is enhanced compared to the endogenous activity, or by strengthening or introducing the *nuo* operon.

Since the *nuo* operon of the present disclosure reduces NADH accumulation due to the enhancement of the OPS biosynthetic pathway, a microorganism that has been modified to enhance the activity of the NADH:quinone oxidoreductase; or a microorganism into which the *nuo* operon is introduced may have the property of increasing the OPS production and thus can be useful for the production of OPS.

The *nuo* operon may include a nucleotide sequence encoding an amino acid sequence having a homology or identity of at least 70%, 80%, 85%, 90%, 95%, 96%, 97%, 98%, or 99% or more to the amino acid sequences represented by SEQ ID NOS: 45 to 57. Specifically, the *nuo* operon may include a structure gene sequence encoding the amino acid sequences of SEQ ID NOS: 45 to 57, or amino acid sequences having a homology or identity thereto and exhibiting the corresponding function; and an expression regulatory region that regulates the expression of the sequence of the structure gene. The sequences of SEQ ID NOS: 45 to 57 can be confirmed in Genbank of NCBI, a known database.

Specifically, the *nuo* operon may be a nucleotide sequence of SEQ ID NO: 3 and/or a nucleotide sequence having a homology or identity of at least 70%, 80%, 85%, 90%, 95%, 96%, 97%, 98%, or 99% or more to SEQ ID NO: 3. Additionally, it is apparent that any nucleotide sequence, in which part of the sequence is deleted, modified, substituted, or added, may also fall within the scope of the present disclosure as long as the nucleotide sequence has such a homology or identity and exhibits a function corresponding to that of the *nuo* operon.

In any one of the above-described embodiments, the recombinant microorganism of the present disclosure may be a microorganism in which the SerB activity is attenuated.

The "SerB" of the present disclosure has an activity of converting OPS to L-serine, and thus the microorganism modified to weaken the SerB activity has the property of accumulating OPS therein, which may be useful for the production of OPS. The SerB of the present disclosure may be a protein having or including the amino acid sequence represented by SEQ ID NO: 4, or may be a protein consisting of or consisting essentially of the amino acid sequence represented by SEQ ID NO: 4, but is not limited thereto. Additionally, the SerB of the present disclosure may have or include an amino acid sequence having a homology or identity of at least 70%, 80%, 90%, 95% or 99% or more to the amino acid sequence represented by SEQ ID NO: 4, as long as it shows the SerB activity. Moreover, the SerB of the present disclosure may consist of or consist essentially of an amino acid sequence having a homology or identity of at least 70%, 80%, 90%, 95% or 99% or more to the amino acid sequence represented by SEQ ID NO: 4, but is not limited thereto. In addition, the polynucleotide encoding the SerB may have or include a nucleotide sequence encoding the amino acid sequence represented by SEQ ID NO: 4. Further, the polynucleotide encoding the SerB may consist of or consist essentially of a nucleotide sequence encoding the amino acid sequence represented by SEQ ID NO: 4 The polynucleotide encoding the SerB of the present disclosure may undergo various modifications in the coding region within the scope that does not change the amino acid sequence of the SerB protein, due to codon degeneracy or in consideration of the codons preferred in an organism in which the SerB protein is to be expressed. The polynucleotide encoding SerB of the present disclosure may have or include a nucleotide sequence having a homology or identity of at least 70%, 80%, 90%, 95% or 99% or more, and less than 100% to the nucleotide sequence of SEQ ID NO: 5. Additionally, the polynucleotide encoding SerB of the present disclosure may consist of or consist essentially of a nucleotide sequence having a homology or identity of at least 70%, 80%, 90%, 95% or 99% or more, and less than 100% to the nucleotide sequence of SEQ ID NO: 5, but is not limited thereto.

In any one of the above-described embodiments, the recombinant microorganism of the present disclosure may be a microorganism in which the activity of O-phosphoserine export protein is enhanced. For example, it may be a microorganism in which the YhhS activity is enhanced.

The "YhhS" of the present disclosure has an activity of exporting OPS, and thus the microorganism modified to enhance the YhhS activity has the property of exporting OPS, which may be useful for the production of OPS. The YhhS of the present disclosure may be a protein having or including an amino acid sequence represented by SEQ ID NO: 6, or may be a protein consisting of or consisting essentially of an amino acid sequence represented by SEQ ID NO: 6, but is not limited thereto. Additionally, the YhhS of the present disclosure may have or include an amino acid sequence having a homology or identity of at least 70%, 80%, 90%, 95% or 99% or more to the amino acid sequence represented by SEQ ID NO: 6, as long as it shows the YhhS activity. Moreover, the YhhS of the present disclosure may consist of or consist essentially of an amino acid sequence having a homology or identity of at least 70%, 80%, 90%, 95% or 99% or more to the amino acid sequence represented by SEQ ID NO: 6, but is not limited thereto. In addition, the polynucleotide encoding the YhhS may have or include a nucleotide sequence encoding the amino acid sequence represented by SEQ ID NO: 6. Further, the polynucleotide encoding the YhhS may consist of or consist essentially of a nucleotide sequence encoding the amino acid sequence represented by SEQ ID NO: 6. The polynucleotide encoding YhhS of the present disclosure may undergo various modifications in the coding region within the scope that does not change the amino acid sequence of the YhhS protein, due to codon degeneracy or in consideration of the codons preferred in an organism in which the YhhS protein is to be expressed. The polynucleotide encoding YhhS of the present disclosure may have or include a nucleotide sequence having a homology or identity of at least 70%, 80%, 90%, 95% or 99% or more, and less than 100% to the nucleotide sequence of SEQ ID NO: 7. Additionally, the polynucleotide encoding YhhS of the present disclosure may consist of or consist essentially of a nucleotide sequence having a homology or identity of at least 70%, 80%, 90%, 95% or 99% or more, and less than 100% to the nucleotide sequence of SEQ ID NO: 7, but is not limited thereto.

In one embodiment, the microorganism including modifications to enhance the ability to produce OPS and/or the ability to export the OPS out of the cell; or to enhance the OPS-decomposing and/or importing ability may be CA07-0012 (KCCM 11121P; US2012-0190081A), CA07-4821, or CA07-4828, but is not limited thereto. Regarding the contents of the OPS-producing microorganism, the disclosures in KR Patent Publication No. 1381048 or US Application Publication No. 2012-0190081, *etc*. may be used as references of the present disclosure, in addition to those described above, but are not limited thereto.

Another aspect of the present disclosure provides a method for producing O-phosphoserine, including culturing an O-phosphoserine-producing recombinant microorganism, in which the activity of MntH protein is enhanced compared to the endogenous activity, in a medium.

The MntH protein, endogenous activity, enhancement, O-phosphoserine, and microorganism are as described above.

As used herein, the term "cultivation" means that the microorganism is grown under appropriately controlled environmental conditions. The cultivation process of the present disclosure may be performed in a suitable culture medium and culture conditions known in the art. Such a cultivation process may be easily adjusted for use by those skilled in the art according to the strain to be selected. Specifically, the cultivation may be a batch culture, a continuous culture, or a fed-batch culture, but is not limited thereto.

In culturing the microorganism, the medium may further contain glycine or serine. Glycine may be provided in the form of purified glycine, a glycine-containing yeast extract, or tryptone. The concentration of glycine to be contained in the medium is generally 0.1 g/L to 10 g/L, and specifically 0.5 g/L to 3 g/L. Additionally, serine may be provided in the form of purified serine, a serine-containing yeast extract, or tryptone. The concentration of serine to be contained in the medium is generally 0.1 g/L to 5 g/L, and specifically 0.1 g/L to 1 g/L.

Examples of the carbon source to be contained in the medium may include saccharides and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These carbon sources may be used alone or in combination, but are not limited thereto.

Examples of the nitrogen source to be contained in the medium may include organic nitrogen sources such as peptone, yeast extract, meat gravy, malt extract, corn steep liquor, and bean flour; and inorganic nitrogen sources such as urea, ammonium sulfate, ammonium chloride, ammonium phosphate, ammonium carbonate, and ammonium nitrate. These nitrogen sources may be used alone or in combination, but are not limited thereto.

Examples of the phosphorous source to be contained in the medium may include potassium dihydrogen phosphate, dipotassium hydrogen phosphate, and corresponding sodium-containing salts, but are not limited thereto.

Additionally, the culture media may include metal salts, such as magnesium sulfate or iron sulfate, and may further contain amino acids, vitamins and appropriate precursors. These culture media or precursors may be added to the culture in the form of a batch culture or continuous culture, but are not limited thereto.

The pH of the culture may be adjusted by adding a compound such as ammonium hydroxide, potassium hydroxide, ammonia, phosphoric acid, and sulfuric acid during cultivation in an appropriate manner. Additionally, bubble formation may be prevented during the cultivation using an antifoaming agent such as fatty acid polyglycol ester. Further, oxygen gas or a gas containing oxygen may be injected to the culture in order to maintain aerobic conditions of the culture; or nitrogen gas, hydrogen gas, or carbon dioxide may be injected to maintain anaerobic or microaerobic conditions, without the injection of gas. The temperature of the culture may be in the range from 25°C to 40°C and specifically from 30°C to 35°C. The cultivation may be continued until the production of a useful material can be obtained, specifically for from 10 hours to 100 hours, but is not limited to these illustrative examples.

The method for producing OPS of the present disclosure may further include a step of preparing the microorganism of the present disclosure, a step of preparing a medium for culturing the microorganism, or a combination thereof (regardless of the order, in any order), for example, prior to the culturing step.

The method for producing OPS of the present disclosure may further include a step of recovering OPS from the cultured medium (medium on which the culture was grown) or the cultured microorganism. The recovering step may be further included after the culturing step.

In the recovering step, the desired OPS may be collected using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC or a combination thereof may be used, and the desired OPS can be recovered from the medium or the microorganisms using suitable methods known in the art.

Further, the method for producing OPS of the present disclosure may further include a purification step, which may be performed using an appropriate method known in the art. In one example, when the method for producing OPS of the present disclosure includes both a recovering step and a purification step, the recovering step and the purification step may be performed continuously or intermittently regardless of the order or simultaneously, or may be integrated into one step, but the method is not limited thereto. Thus, the recovered OPS may be in a purified state or a microbial fermentation broth containing OPS. In addition, the recovery of OPS can be performed efficiently by adding a suitable method known in the art before and after the culturing step and before and after the recovering step.

Still another aspect of the present disclosure provides a method for producing cysteine or a derivative thereof, including:
a) culturing an O-phosphoserine-producing recombinant microorganism, in which the activity of MntH protein is enhanced compared to the endogenous activity, in a medium to produce O-phosphoserine or a medium containing O-phosphoserine; and
b) reacting O-phosphoserine produced in step a) or a medium containing the same with a sulfide in the presence of O-phosphoserine sulfhydrylase (OPSS) or a microorganism including the same.

The steps a) and b) are not necessarily limited by the order, *i.e*., to be performed continuously or sequentially, and there is no time interval between these steps, and the steps may be performed simultaneously, or with an interval of several seconds, several minutes, several hours, several days.

The MntH protein, endogenous activity, enhancement, O-phosphoserine (OPS), and microorganism are as described above.

As used herein, the term "derivative" refers to similar compounds obtained by chemically modifying a portion of any compound. The term usually refers to compounds in which a hydrogen atom or a particular atom group is substituted with another atom or atom group.

As used herein, the term "cysteine derivative" refers to compounds in which a hydrogen atom or a particular atom group in cysteine is substituted with another atom or atom group. For example, the cysteine derivatives may have a form in which the nitrogen atom of the amine group (-NH₂) or the sulfur atom of the thiol group (-SH) in cysteine has another atom or atom group attached thereto, and the examples of cysteine derivatives may include N-acetylcysteine (NAC), S-Carboxymetylcysteine (SCMC), BOC-CYS(ME)-OH,(R)-S-(2-Amino-2-carboxyethyl)-L-homocysteine, (R)-2-Amino-3-sulfopropionic acid, D-2-Amino-4-(ethylthio)butyric acid, 3-sulfino-L-alanine, Fmoc-Cys(Boc-methyl)-OH, Seleno-L-cystine, S-(2-Thiazolyl)-L-cysteine, S-(2-Thienyl)-L-cysteine, S-(4-Tolyl)-L-cysteine, *etc.,* but are not limited thereto.

As long as cysteine is produced according to the method of the present disclosure, conversion to cysteine derivatives may be easily converted into various cysteine derivatives by a method well known in the art.

In the present disclosure, the method for producing cysteine derivatives may further include converting the cysteine produced in step b) into a cysteine derivative.

Specifically, in the present disclosure, the method for producing cysteine derivatives may include the steps of: producing cysteine according to the method of the present disclosure described above; and converting the produced cysteine into a cysteine derivative.

The conversion of the produced cysteine into a cysteine derivative may be performed by a method well known in the art, for example, cysteine may be synthesized into N-acetylcysteine (NAC) by a reaction with an acetylation agent, or may be synthesized into S-carboxymethylcysteine (SCMC) by a reaction with a haloacetic acid in basic conditions according to methods known in the art, but the examples are not limited thereto.

These cysteine derivatives are used mainly as pharmaceutical materials for antitussive agents, cough-relieving agents, and therapeutic agents for bronchitis, bronchial asthma, laryngopharyngitis, *etc.,* but are not limited thereto.

As used herein, the term "O-phosphoserine sulfhydrylase (OPSS)" refers to an enzyme that catalyzes a reaction by which OPS is converted into cysteine by providing from a thiol group (SH group) to OPS. The enzyme may have been first found in *Aeropyrum pernix, Mycobacterium tuberculosis, Mycobacterium smegmatics,* and *Trichomonas vaginalis* (Mino K and Ishikawa K, FEBS Letters, 551: 133-138, 2003; Burns K E et al., J. Am. Chem. Soc., 127: 11602-11603, 2005). Additionally, the OPSS may include not only wild-type OPSS proteins, but also variant proteins, in which a part of the sequence is deleted, substituted, or added in the polynucleotide sequence encoding the OPSS, which show activity that is equal to or higher than the biological activity of the wild-type OPSS proteins, and may also include all OPSS proteins disclosed in Korean Application Publication No. 2012-0041115 and KR Patent Publication No. 1208267, and their variant proteins.

The sulfide may be any sulfide provided not only in a solid form generally used in the art, but also in a liquid or gas form due to the difference in pH, pressure, and solubility, and thus can be converted to a thiol (SH) group in the form of sulfide (S²⁻), thiosulfate (S₂O₃²⁻), *etc.*, without limitation. Specifically, the sulfide may include Na₂S, NaSH, (NH₄)₂S, H₂S or Na₂S₂O₃, which can provide a thiol group to OPS, but is not limited thereto. In the reaction, a single thiol group is supplied to a single reactive OPS group to produce a single cysteine or a derivative thereof. Herein, a sulfide may be added in an amount of 0.1 to 3 molar equivalents, and specifically 1 to 2 molar equivalents based on the molar concentration of OPS, but is not limited thereto.

In addition, the method for producing cysteine derivatives may further include a step of recovering the cysteine produced by the reaction step. Herein, the desired cysteine may be collected by isolating and purifying cysteine from the reaction solution using a suitable reaction known in the art.

Yet another aspect of the present disclosure provides a composition for producing O-phosphoserine, cysteine or cysteine derivatives, including: an O-phosphoserine-producing recombinant microorganism, in which the activity of MntH protein is enhanced compared to the endogenous activity; a medium on which the microorganism is cultured; or a combination thereof.

The composition of the present disclosure may further include any suitable excipient commonly used in compositions for producing O-phosphoserine, cysteine or cysteine derivatives, and such excipients may include, for example, preservatives, wetting agents, dispersing agents, suspending agents, buffers, stabilizers, or isotonic agents, *etc.,* but are not limited thereto.

Even another aspect of the present disclosure provides a method for producing an O-phosphoserine-producing recombinant microorganism, including enhancing the activity of MntH protein compared to the endogenous activity.

Further another aspect of the present disclosure provides the use of an O-phosphoserine-producing recombinant microorganism, in which the activity of MntH protein is enhanced compared to the endogenous activity, in the production of O-phosphoserine, cysteine or cysteine derivatives.

The MntH protein, endogenous activity, enhancement, O-phosphoserine (OPS), cysteine, cysteine derivatives, and microorganism of the aspect are as described above.

### [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

### Example 1: Preparation of O-Phosphoserine (OPS)-Producing Strain with Enhanced YhhS Expression

### 1-1. Preparation of Vector for Enhancing YhhS Expression

PCR was performed to obtain gene fragments in the upstream region of the wild-type promoter of the *yhhS* gene (SEQ ID NO: 7), where homologous recombination occurs on the chromosomes, using a primer pair of SEQ ID NOS: 10 and 11, and gene fragments in the downstream region of the wild-type promoter of the *yhhS* gene using a primer pair of SEQ ID NOS: 14 and 15 based on the wild-type *E*. *coli* ATCC27325 chromosomal DNA as a template. In addition, PCR was performed to obtain the Ptrc promoter (SEQ ID N: 59) using a primer pair of SEQ ID NOS: 12 and 13 based on the pCL_Ptrc-gfp (WO 2016-024771 A1) as a template.

Solg^{™} Pfu-X DNA polymerase was used for the PCR, and the PCR was performed under PCR amplification conditions of denaturation at 95°C for 2 minutes, followed by 30 cycles of denaturation at 95°C for 30 seconds, annealing at 60°C for 30 seconds, and polymerization at 72°C for 60 seconds, and then polymerization at 72°C for 5 minutes.

The upstream fragments and downstream fragments of the *yhhS* promoter, and the Ptrc promoter fragments obtained by the above process were cloned with the pSKH130 vector (SEQ ID NO: 58, U.S. Application Publication No. 2020-0048619) for chromosomal transformation after being cleaved with the restriction enzyme EcoRV using an in-fusion cloning kit (Clontech Laboratories, Inc.) to obtain a recombinant plasmid, and it was named pSKH_Ptrc-yhhS.

The primer sequences used above are shown in Table 1.

**[Table 1]**

| SEQ ID NO: | Sequence Name | Sequence (5' -> 3') |
|---|---|---|
| 10 | yhhS UP F | CTGCAGGAATTCGATGAAGGTAACCACTTTTTCCG |
| 11 | yhhS UP R | GAGTTGCAGCAAGCGGAGGATCACCACATTTTTAC |
| 12 | Ptrc F | AATGTGGTGATCCTCCGCTTGCTGCAACTCTCTCA |
| 13 | Ptrc R | TACGGGTTCGGGCATGATAGCTCTCCTGTGTGAAA |
| 14 | yhhS Down F | CACAGGAGAGCTATCATGCCCGAACCCGTAGCCGA |
| 15 | yhhS Down R | GTCGACTAGCGTGATAGCGGTTTGCCTTTACTGGC |

### 1-2. Preparation of Strain with Enhanced YhhS Expression

The pSKH_Ptrc-yhhS prepared in Example 1-1 was introduced into the CA07-0012 (KCCM 11121P, U.S. Patent Publication No. US 8557549 B2), which is an OPS-producing strain, in which the OPS-decomposing ability has been weakened by deleting the endogenous phosphoserine phosphatase (SerB) in the wild-type *E. coli* K-12 W3110, to enhance the expression of YhhS (SEQ ID NO: 6), which is a protein having an OPS-exporting ability, thereby further enhancing the OPS-exporting ability.

The pSKH_Ptrc-yhhS prepared in Example 1-1 was transformed into the CA07-0012 strain by electroporation (Appl. Microbiol.Biotechnol. (1999) 52:541-545) and then the desired strain was obtained using R6K and kanamycin at the first crossover. Subsequently, the strain was subjected to a secondary crossover in a medium containing sucrose to obtain a strain in which the kanamycin resistance gene was deleted and the nucleotide sequence of the Ptrc promoter was inserted at the terminal of the nucleotide sequence of the wild-type promoter of the *yhhS* gene. The insertion of the nucleotide sequence of the Ptrc promoter was confirmed through genome sequencing and PCR amplification using a primer pair of SEQ ID NOS: 16 and 17, which can amplify the external region of the upstream region and downstream region of the homologous recombination, respectively. The thus-obtained strain was named CA07-4821(CA07-0012ΔPn_yhhS::Ptrc_yhhS).

The primer sequences used above are shown in Table 2.

**[Table 2]**

| SEQ ID NO: | Sequence Name | Sequence (5' -> 3') |
|---|---|---|
| 16 | yhhSP Conf F | AGTTGAGCAAAAACGCCAGT |
| 17 | yhhSP Conf R | GCCATACCGTACAGCCAGAC |

### Example 2: Preparation of OPS-Producing Strain Introduced with nuo Operon

### 2-1. Preparation of Vector for Introducing nuo Operon

PCR was performed to obtain gene fragments in the upstream region of the wild-type promoter of the *nuo* operon (SEQ ID NO: 3) using a primer pair of SEQ ID NOS: 18 and 19, and gene fragments in the downstream region of the wild-type promoter of the *nuo* operon using a primer pair of SEQ ID NOS: 20 and 21 based on the wild-type *E*. *coli* ATCC27325 chromosomal DNA as a template. In addition, PCR was performed to obtain the promoter region of the *rmf* gene (SEQ ID NO: 8) using a primer pair of SEQ ID NOS: 22 and 23 based on the wild-type *E. coli* ATCC27325 chromosomal DNA as a template.

Solg^{™} Pfu-X DNA polymerase was used for the PCR, and the PCR was performed under PCR amplification conditions of denaturation at 95°C for 2 minutes, followed by 30 cycles of denaturation at 95°C for 30 seconds, annealing at 60°C for 30 seconds, and polymerization at 72°C for 60 seconds, and then polymerization at 72°C for 5 minutes.

The upstream fragments and downstream fragments of the *nuo* promoter, and the *rmf* promoter fragments obtained by the above process were cloned with the pSKH130 for chromosomal transformation after being cleaved with the restriction enzyme EcoRV using an in-fusion cloning kit to obtain a recombinant plasmid, and it was named pSKH_Prmf-nuoA.

The primer sequences used above are shown in Table 3.

**[Table 3]**

| SEQ ID NO: | Sequence Name | Sequence (5' -> 3') |
|---|---|---|
| 18 | nuoA UP F | CTGCAGGAATTCGATCAACCACCAGAGATTCACGT |
| 19 | nuoA UP R | GCTTCCTGACTCCAGTGCTTACTCATCAAAAGTAG |
| 20 | nuoA Down F | TGAGGGAAACGAGGCATGAGTATGTCAACATCCAC |
| 21 | nuoA Down R | ACGGTGAGAACGACCGTTACATCACGCTAGTCGAC |
| 22 | Prmf F2 | TTTGATGAGTAAGCACTGGAGTCAGGAAGCCGCTT |
| 23 | Prmf R2 | TGTTGACATACTCATGCCTCGTTTCCCTCATACTG |

### 2-2. Preparation of Strain Introduced with nuo Operon

The pSKH_Prmf-nuoA prepared in Example 2-1 was transformed into the CA07-4821 strain, which is an OPS-producing strain in which OPS-decomposing ability has been weakened by deleting the endogenous phosphoserine phosphatase (SerB), by electroporation and then the desired strain was obtained using R6K and kanamycin at the first crossover. Subsequently, the strain was subjected to a secondary crossover in a medium containing sucrose to obtain a strain in which the kanamycin resistance gene was deleted and the promoter nucleotide sequence of the *rmf* gene was inserted at the terminal of the nucleotide sequence of the wild-type promoter of the *nuo* gene. The insertion of the nucleotide sequence of the *rmf* promoter was confirmed through genome sequencing and PCR amplification using a primer pair of SEQ ID NOS: 24 and 25, which can amplify the external region of the upstream region and downstream region of the homologous recombination, respectively. The thus-obtained strain was named CA07-4828(CA07-4821ΔPn_nuoA::Prmf_nuoA).

The primer sequences used above are shown in Table 4 below.

**[Table 4]**

| SEQ ID NO: | Sequence Name | Sequence (5' -> 3') |
|---|---|---|
| 24 | nuo Con F | GAGAGCTACCATAATCCGTG |
| 25 | nuo Con R | CATCTCAACGTAACAGCAGG |

### Example 3: Preparation of Strains with Enhanced or Deleted MntH Expression

### 3-1. Preparation of Vectors for Enhancing MntH Expression

As a result of the previous study, it was found that the average transcription levels of the *mntH, serC*, and *rmf* genes in the OPS-producing host strains were 698, 6215, and 32205, respectively, as shown in Table 5 below, thereby confirming that the average transcription level of the *rmf* gene was 46 times higher and the average transcription level of the *serC* gene was 8.9 times higher as compared to the average transcription level of the *mntH* gene. Accordingly, it was confirmed that the *rmf* gene promoter and the *serC* gene promoter were relatively stronger promoters, compared to the *mntH* promoter.

**[Table 5]**

| Gene name | Early Exponential | Mid Exponential | Late Exponential | Stationary | Average |
|---|---|---|---|---|---|
| *mntH* | 670 | 740 | 792 | 589 | 698 |
| *serC* | 5385 | 5485 | 5338 | 8652 | 6215 |
| *rmf* | 30725 | 28192 | 28109 | 41792 | 32205 |

Thus, strains with enhanced MntH expression were prepared by further inserting the *rmf* promoter and *serC* promoter (SEQ ID NO: 9), which were found to show stronger activity, at the terminal of the promoter of the *mntH* gene (SEQ ID NO: 2) in the OPS-producing microorganism.

PCR was performed to obtain gene fragments in the upstream region of the wild-type promoter of the *mntH* gene using a primer pair of SEQ ID NOS: 26 and 27, and gene fragments in the downstream region of the wild-type promoter of the *mntH* gene using a primer pair of SEQ ID NOS: 28 and 29 based on the wild-type *E. coli* ATCC27325 chromosomal DNA as a template. In addition, PCR was performed to obtain the promoter region of the *rmf* gene using a primer pair of SEQ ID NOS: 30 and 31 based on the wild-type *E. coli* ATCC27325 chromosomal DNA as a template.

Solg^{™} Pfu-X DNA polymerase was used for the PCR, and the PCR was performed under PCR amplification conditions of denaturation at 95°C for 2 minutes, followed by 30 cycles of denaturation at 95°C for 30 seconds, annealing at 60°C for 30 seconds, and polymerization at 72°C for 60 seconds, and then polymerization at 72°C for 5 minutes.

The upstream fragments and downstream fragments of the *mntH* promoter, and the *rmf* promoter fragments obtained by the above process were cloned with the pSKH130 for chromosomal transformation after being cleaved with the restriction enzyme EcoRV using an in-fusion cloning kit to obtain a recombinant plasmid, and it was named pSKH130_Prmf-mntH.

PCR was performed to obtain gene fragments in the upstream region of the wild-type promoter of the *mntH* gene using a primer pair of SEQ ID NOS: 32 and 33, and gene fragments in the downstream region of the wild-type promoter of the *mntH* gene using a primer pair of SEQ ID NOS: 34 and 35 based on the wild-type *E. coli* ATCC27325 chromosomal DNA as a template. In addition, PCR was performed to obtain the promoter region of the *serC* gene using a primer pair of SEQ ID NOS: 36 and 37 based on the wild-type *E. coli* ATCC27325 chromosomal DNA as a template.

Solg^{™} Pfu-X DNA polymerase was used for the PCR, and the PCR was performed under PCR amplification conditions of denaturation at 95°C for 2 minutes, followed by 30 cycles of denaturation at 95°C for 30 seconds, annealing at 60°C for 30 seconds, and polymerization at 72°C for 60 seconds, and then polymerization at 72°C for 5 minutes.

The upstream fragments and downstream fragments of the *mntH* promoter, and the *serC* promoter fragments obtained by the above process were cloned with the pSKH130 for chromosomal transformation after being cleaved with the restriction enzyme EcoRV using an in-fusion cloning kit to obtain a recombinant plasmid, and it was named pSKH130_PserC-mntH.

### 3-2. Preparation of Vector for Deleting MntH

PCR was performed to obtain gene fragments in the upstream region of the wild-type promoter of the *mntH* gene using a primer pair of SEQ ID NOS: 38 and 39, and gene fragments in the downstream region of the wild-type promoter of the *mntH* gene using a primer pair of SEQ ID NOS: 40 and 41 based on the wild-type *E. coli* ATCC27325 chromosomal DNA as a template.

PCR was performed in the same manner as in Example 3-1 to obtain the upstream and downstream fragments of the *mntH* promoter, and the fragments were cloned in the same manner as in Example 3-1 to obtain a recombinant plasmid. The thus-obtained plasmid was named pSKH_ΔmntH.

The primer sequences used in Examples 3-1 and 3-2 are shown in Table 6 below.

**[Table 6]**

| SEQ ID NO: | Sequence Name | Sequence (5' -> 3') |
|---|---|---|
| 26 | mntH UP F1 | CTGCAGGAATTCgatAAGGATTCCAGTTTGCCCAT |
| 27 | mntH UP R1 | GCAATAGAAGCGGCTTCCTGCTTGTGCCTCTAAAAC |
| 28 | mntH Down F1 | TGAGGGAAACGAGGCATGACGAACTATCGC |
| 29 | mntH Down R1 | GTCGACTAGCGTgatGACAAAACCGGCAATAGTCA |
| 30 | Prmf F1 | TTTTAGAGGCACAAGCAGGAAGCCGCTTCTATTGC |
| 31 | Prmf R1 | GCGATAGTTCGTCATGCCTCGTTTCCCTCATACTG |
| 32 | mntH UP F2 | cTGCAGGAATTCGATCGTTAAAGGATTCCAGTTTG |
| 33 | mntH UP R2 | ACAACGAATACAGGCCTTGTGCCTCTAAAACATAG |
| 34 | mntH Down F2 | TTTTAGAGGCACAAGGCCTGTATTCGTTGTAGTGA |
| 35 | mntH Down R2 | GCGATAGTTCGTCATTTCCCCTCACCACGTTGCGT |
| 36 | PserC F | ACGTGGTGAGGGGAAATGACGAACTATCGCGTTGA |
| 37 | PserC R | GTCGACTAGCGTGATATTGACAAAACCGGCAATAG |
| 38 | mntH del F1 | CTGCAGGAATTCgatAAAGGATTCCAGTTTGCCCA |
| 39 | mntH del R1 | GCGACGCTCATTCAACTTGTGCCTCTAAAACATAGC C |
| 40 | mntH del F2 | GTTTTAGAGGCACAAGTTGAATGAGCGTCGCATCTG |
| 41 | mntH del R2 | GTCGACTAGCGTgatGGTTAAGTCTTTTTATGGCGG |

### 3-3. Preparation of Strains with Enhanced MntH Expression

The pSKH_PserC-mntH prepared in Example 3-1 was transformed into the CA07-4821 strain of Example 1-2, which is an OPS-producing strain with enhanced YhhS expression, by electroporation and then subjected to a secondary crossover to obtain a strain in which the promoter nucleotide sequence of the *serC* gene was inserted at the terminal of the nucleotide sequence of the wild-type promoter of the *mntH* gene. The insertion of the nucleotide sequence of the *serC* promoter was confirmed through genome sequencing and PCR amplification using a primer pair of SEQ ID NOS: 42 and 43, which can amplify the external region of the upstream region and downstream region of the homologous recombination, respectively. The thus-obtained strain was named CA07-4895(CA07-4821ΔPn_mntH::PserC_mntH).

The pSKH_Prmf-mntH and pSKH_PserC-mntH prepared in Example 3-1 were each transformed into the CA07-4828 strain of Example 2-2, which is an OPS-producing strain introduced with *nuo* operon, by electroporation and then subjected to a secondary crossover to obtain strains in which the promoter nucleotide sequence of the *serC* or *rmf* gene was inserted at the terminal of the nucleotide sequence of the wild-type promoter of the *mntH* gene, respectively. The insertion of the nucleotide sequence of the *serC* or *rmf* promoter was confirmed through genome sequencing and PCR amplification using a primer pair of SEQ ID NOS: 42 and 43, which can amplify the external region of the upstream region and downstream region of the homologous recombination, respectively. The thus-obtained strains were named CA07-4898(CA07-4828ΔPn_mntH::PserC_mntH) and CA07-4899(CA07-4828ΔPn_mntH::Prmf_mntH), respectively.

### 3-4. Preparation of Strain with Deleted MntH

The pSKH_ΔmntH prepared in Example 3-2 was transformed into the CA07-4828 strain of Example 2-2, which is an OPS-producing strain introduced with *nuo* operon, by electroporation and then subjected to a secondary crossover to obtain a strain with deleted *mntH* gene. The deletion of the *mntH* nucleotide sequence was confirmed through genome sequencing and PCR amplification using a primer pair of SEQ ID NOS: 42 and 44, which can amplify the external region of the upstream region and downstream region of the homologous recombination, respectively. The thus-obtained strain was named CA07-4897(CA07-4828ΔmntH).

The primer sequences used in Examples 3-3 and 3-4 are shown in Table 7 below.

**[Table 7]**

| SEQ ID NO: | Sequence Name | Sequence (5' -> 3') |
|---|---|---|
| 42 | mntH Conf F | TCATACATTTCTGCTATGTT |
| 43 | mntH Conf R | AGAGGATCTGAATCAGCATC |
| 44 | mntH Conf R2 | GTTTCTTGCGCTATTCTCGG |

### Example 4: Evaluation of OPS-Producing Ability of Strains

In order to measure the OPS-producing ability of the CA07-4895 strain, CA07-4898 strain and CA07-4899 strain of Example 3-3, which are strains with enhanced MntH expression, the CA07-4897 strain of Example 3-4, which is a strain with deleted MntH, and the CA07-0012 strain of Example 1-2, which is the parent strain, the CA07-4821 strain of Example 1-2, and the CA07-4828 strain of Example 2-2, which are control strains, flask fermentation titer evaluation was performed.

Each of the strains was plated out on a solid LB medium and cultured in a 33°C incubator overnight. The strains cultured in the solid LB medium overnight were inoculated into 25 mL of the following titer medium and then cultured in an incubator at a rate of 200 rpm for 48 hours at 33°C. After completion of the culture, the concentration of OPS was measured using HPLC and the results are shown in Table 8 below.

### <Titer Medium>

Glucose 40g/L, KH₂PO₄ 6g/L, (NH₄)2SO₄ 17g/L, MgSO₄·7H₂O 1g/L, MnSO₄·4H₂O 5mg/L, FeSO₄·7H₂O 10mg/L, L-glycine 1.5g/L, Yeast Extract 2.5g/L, CaCO₃ 30g/L, pH 6.8

**[Table 8]**

| Name of Strains | Parent Strains | Gene Type | OPS concentration (g/L) |
|---|---|---|---|
| CA07-0012 | W3110 | W3110ΔserB | 5.54 |
| CA07-4821 | CA07-0012 | ΔPn_yhhS::Ptrc_yhhS | 7.59 |
| CA07-4895 | CA07-4821 | ΔPn_mntH::PserC_mnt H | 8.30 |
| CA07-4828 | CA07-4821 | ΔPn_nuoA::Prmf nuoA | 8.19 |
| CA07-4898 | CA07-4828 | ΔPn_mntH::PserC_mnt H | 8.38 |
| CA07-4899 | CA07-4828 | ΔPn_mntH::Prmf mntH | 8.62 |
| CA07-4897 | CA07-4828 | ΔmntH | 7.35 |

As shown in Table 8, the MntH-enhanced CA07-4895 strain, which used the YhhS-enhanced CA07-4821 strain as the parent strain, showed an increased OPS-producing ability by 9.4% compared to the parent strain.

The CA07-4828 strain, in which the YhhS and *nuo* operons were simultaneously enhanced, showed an increased OPS-producing ability by 7.9% compared to the YhhS-enhanced CA07-4821 strain.

Additionally, the MntH-enhanced CA07-4898 and CA07-4899 strains, which used CA07-4828 with enhanced *nuo* operon as the parent strain, showed an increased OPS-producing ability by 2.3% and 5.3%, respectively, compared to the parent strain. The CA07-4897 strain with deleted MntH showed a decreased OPS-producing ability by -10.3% compared to the parent strain.

Accordingly, it was confirmed that the enhancement of MntH increased OPS-producing ability.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. An O-phosphoserine-producing recombinant microorganism, in which the activity of MntH protein is enhanced compared to the endogenous activity.

2. The microorganism of claim 1, wherein the recombinant microorganism has an enhanced O-phosphoserine-producing ability compared to the endogenous O-phosphoserine-producing ability.

3. The microorganism of claim 1, wherein the MntH protein comprises an amino acid sequence of SEQ ID NO: 1.

4. The microorganism of claim 1, wherein the polynucleotide encoding the MntH protein comprises a nucleotide sequence of SEQ ID NO: 2.

5. The microorganism of claim 1, wherein the activity of one or more selected from NADH:quinone oxidoreductase and O-phosphoserine export protein is further enhanced compared to the endogenous activity.

6. The microorganism of claim 1, wherein the recombinant microorganism belongs to the genus *Escherichia.*

7. A method for producing O-phosphoserine, comprising culturing an O-phosphoserine-producing recombinant microorganism, in which the activity of MntH protein is enhanced compared to the endogenous activity, in a medium.

8. The method of claim 7, wherein the method further comprises recovering O-phosphoserine from the cultured medium or microorganism.

9. The method of claim 7, wherein the MntH protein comprises an amino acid sequence of SEQ ID NO: 1.

10. The method of claim 7, wherein the polynucleotide encoding the MntH protein comprises a nucleotide sequence of SEQ ID NO: 2.

11. A method for producing cysteine or a derivative thereof, comprising:
a) culturing an O-phosphoserine-producing recombinant microorganism, in which the activity of MntH is enhanced compared to the endogenous activity, in a medium to produce O-phosphoserine or a medium containing O-phosphoserine; and
b) reacting O-phosphoserine produced in step a) or a medium containing the same with a sulfide in the presence of O-phosphoserine sulfhydrylase (OPSS) or a microorganism comprising the same.

12. The method of claim 11, wherein the sulfide is one or more selected from the group consisting of Na₂S, NaSH, (NH₄)₂S, H₂S and Na₂S₂O₃.

13. The method of claim 11, wherein the MntH protein comprises an amino acid sequence of SEQ ID NO: 1.

14. The method of claim 11, wherein the polynucleotide encoding the MntH protein comprises a nucleotide sequence of SEQ ID NO: 2.

15. Use of an O-phosphoserine-producing recombinant microorganism, in which the activity of MntH protein is enhanced compared to the endogenous activity, in the production of O-phosphoserine, cysteine or cysteine derivatives.
